(19) Europäisches Patentamt
European Patent Office
Office européen des brevets

(11) Publication number: **0 574 433 B1**

# (12) EUROPEAN PATENT SPECIFICATION

(45) Date of publication of patent specification: **31.05.95**

(51) Int. Cl.$^6$: **A61K 31/495**, C07D 237/26, C07D 237/30, C07D 403/12

(21) Application number: **92905252.0**

(22) Date of filing: **02.03.92**

(86) International application number: **PCT/EP92/00454**

(87) International publication number: **WO 92/15307 (17.09.92 92/24)**

## (54) 6,9-BIS(AMINO SUBSTITUTED)BENZO g]PHTHALAZINE-5,10-DIONES AS ANTITUMOR AGENTS.

(30) Priority: **08.03.91 IT MI91060**

(43) Date of publication of application: **22.12.93 Bulletin 93/51**

(45) Publication of the grant of the patent: **31.05.95 Bulletin 95/22**

(84) Designated Contracting States: **AT BE CH DE DK ES FR GB GR IT LI LU NL SE**

(56) References cited:

**Journal of Medicinal Chemistry, vol. 28, no. 8, 1985, Washington US; pages 1124-1126; A.P. KRAPCHO: "Synthesis and antineoplastic evaluations of 5,8-bis (aminoalkyl)amino)-1-azaanthracene-9,10-diones"**

(73) Proprietor: **BOEHRINGER MANNHEIM ITALIA S.P.A.**
**Via S. Uguzzone, 5**
**I-20126 Milano (IT)**

(72) Inventor: **OLIVA, Ambrogio**
**Via S. Uguzzone, 5**
**I-20126 Milano (IT)**
Inventor: **SPINELLI, Silvano**
**Via S. Uguzzone, 5**
**I-20126 Milano (IT)**
Inventor: **MENTA, Ernesto**
**Via S. Uguzzone, 5**
**I-20126 Milano (IT)**
Inventor: **TOGNELLA, Sergio**
**Via S. Uguzzone, 5**
**I-20126 Milano (IT)**

(74) Representative: **Weber, Manfred, Dr. et al**
**c/o Boehringer Mannheim GmbH,**
**Patentabteilung,**
**Sandhoferstrasse 116**
**D-68298 Mannheim (DE)**

EP 0 574 433 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EUROPEAN JOURNAL OF MEDICINAL CHEMISTRY.CHIMICA THERAPEUTICA., vol. 21, no. 2, 1986, Chatenay-Malabry FR; pages 143-149; L. CAMPAYO: "Synthesis and cytostatic activity of 1,4-bis-(alkylamino) benzo (g) phthalazines with complexing properties"**

## Description

Certain 1,4-bis[(aminoalkyl)amino]anthracene-9,10-diones have been reported which show antitumor activity in clinical trials. Of particular interest has been ametantrone, 1,4-bis{[2-(2-hydroxyethylamino)ethyl]-amino]anthracene-9,10-dione and mitoxantrone, 5,8-dihydroxy-1,4-bis{[2-(2-hydroxyethylamino)ethyl]amino]-anthracene-9,10-dione. (Zee-Cheng et al., J. Med. Chem., (1978), 21, 291-4; Cheng et al., "Progress in Medicinal Chemistry", Ellis, G.P. and West, G.B., eds.; Elsevier: Amsterdam, 1983; pp. 20, 83 and references cited therein). Mitoxantrone is a broad spectrum oncolytic agent, whose activity is similar to that of the anthracycline antibiotic doxorubicin. Clinical trials have demonstrated that mitoxantrone has particularly promising activity in the treatment of advanced breast cancer, acute leukemia and lymphoma (Legha, Drugs of Today, (1984), 20, 629). Although animal studies have demonstrated a diminished cardiotoxicity in comparison to doxorubicin, some clinical cardiotoxicity has been observed also with mitoxantrone, mostly in patients previously treated with doxorubicin (R. Stuart Harris et al., Lancet, (1984), 219, and references cited therein).

Ametantrone has been reported to be, in animals, about 10-fold less potent and cardiotoxic than mitoxantrone. Because a delayed toxicity is observed only with mitoxantrone after administration of the two drugs by the i.p. route to non-tumor bearing rats at equieffective antitumor dosages, it is suggested that the presence of the 5,8-dihydroxy substitution in mitoxantrone might be implicated in the delayed deaths (Corbet et al., Cancer Chemother. Pharmacol., (1981), 6, 161).

In addition, both mitoxantrone and ametantrone have a remarkable myelodepressive toxicity and both compounds show cross-resistance to cell hystotypes developing resistance against doxorubicin mediated by overexpression of glycoprotein P. Such a resistance, which is named multidrug resistance, involves a number of antitumor antibiotics, among which amsacrine and podophyllotoxinic derivatives, and it is one of the main reasons for therapeutical failures in the treatment of solid tumors with said antibiotics.

Therefore, search is required for novel anthracenedione antitumor agents, having a higher therapeutical index than mitoxantrone and being effective both in inhibiting or delaying the growth of those solid tumors which are more refractory to chemotherapeutic treatment (such as lung, breast and colon tumors) and against tumor histotypes developing multidrug resistance.

In the search for safer, active analogues of anthracenediones, compounds bearing hydroxysubstituents and/or (aminoalkyl)amino side chains at various positions on the anthracene-9,10-dione nucleus have been studied (C.C. Cheng et al., Drugs of the Future, (1983), 8, 229) without notable improvement.

Aza- and diaza anthracene-9,10-diones such as 6,9-bis(ethoxycarbonylamino)benzo[g]quinoline-5,10-dione (1), 6,9-bis(ethoxycarbonylamino)benzo[g]isoquinoline5,10-dione (2), 6,9-bis(ethoxycarbonylamino)-benzo[g]quinazoline-5,10-dione (3), were disclosed by Potts et al. (Synthesis, (1983, 31). These compounds were reported to be related to the antitumor 1,4-bis[(aminoalkyl)amino]anthracene-9,10-diones; however no antitumor activity data was reported for any of the above compounds. 1-[(aminoalkyl)amino] derivatives (4) of 6,9-dihydroxybenzo[g]isoquinoline-5,10-dione related to mitoxantrone have been disclosed as DNA intercalators but they were completely devoid of any "in vitro" or "in vivo" antitumor activity (Croisy-Delcey et al., Eur. J. Med. Chem., (1988), 23, 101-106).

The 6,9-bis[(aminoalkyl)amino]benzo[g]quinoline-5,10-diones of formulas 5a-d (see Table I) were disclosed in J. Med. Chem. (1985), 28, 1124-26, where they were referred to as 5,8-bis[(aminoakyl)amino]-1-azaanthracenediones.

As reported in table II hereinbelow reported the prior art compounds 5 are clearly less active than the corresponding carbocyclic analogues (6).

In fact, as reported in table II, the compound 5a and 5b are less cytotoxic than the analogues 6a and 6b. Moreover the compound 5a, which is poorly cytotoxic in vitro, is inactive in vivo, and it is both less active and less potent than the carbocyclic analogue 6a.

Even though the introduction of an heteroatom is a common process in the medicinal chemistry, its effect must be evaluated case by case.

In this particular case of aza-analogues of anthracenediones the prior art teachings clearly show that the introduction of a nitrogen atom into the anthracenedione skeleton is detrimental for antitumor activity. In fact the aza-substituted anthracenediones are less cytotoxic than the corresponding anthracenediones and inactive "in vivo".

Thus a person skilled in the art, would have not considered the introduction of heteroatoms into the anthracenedione skeleton as a possible way to obtain more effective antitumor agents.

It is well known that the screening for the discovery of the antitumor agent mitoxantrone (J. Med. Chem., (1978), 2l, 291-4) among a number of analogues has been carried out by using the experimental model of murine leukemia P388: this model is then predictive of antitumor activity in humans at least for

this class of antitumor antibiotics. Many other clinically active antitumor antibiotics are active on murine leukemias P388 and L1210 such as m-amsacrine and doxorubicin.

Moreover mitoxantrone has shown good activity in other significative experimental tumors such as murine Lewis lung carcinoma and MXI human mammary carcinoma.

We have now discovered that the compounds of the invention 6,9-bis[(aminoalkyl)amino]benzo[g]-isoquinoline-5,10-diones are active as antitumor agents: they are highly effective against murine leukemias L1210 and P388 and against Lewis lung carcinoma and MXI human mammary carcinoma.

**Table I**

Prior art compounds

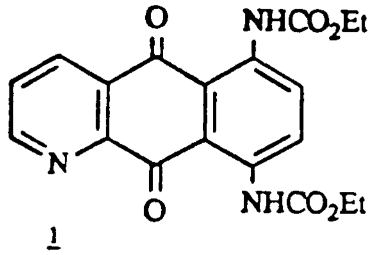

1

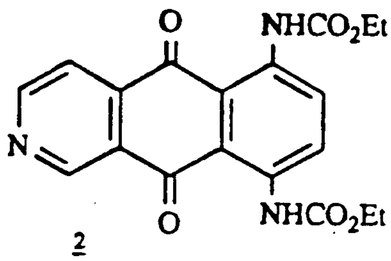

2

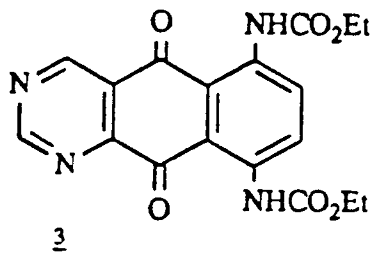

3

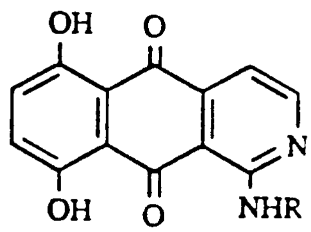

4a R= $CH_2CH_2N(CH_3)_2$

4b R= $CH_2CH_2CH_2N(Et)_2$

4c R= $CH_2CH_2NHCH_2CH_2OH$

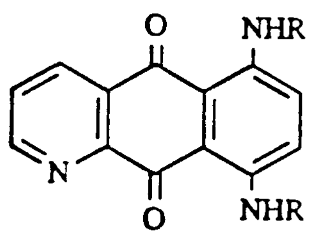

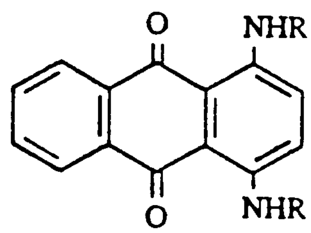

5a R= $CH_2CH_2N(CH_3)_2$

5b R= $CH_2CH_2N(Et)_2$

5c R= $CH_2CH_2CH_2N(CH_3)_2$

5d R= $CH_2CH_2NHCOCH_3$

6a R= $CH_2CH_2N(CH_3)_2$

6b R= $CH_2CH_2N(Et)_2$

Table II

| Antitumor activity of prior art compounds 5 and 6 against murine L 1210 leukemia (from J. Med. Chem. (1985), 28, 1124-1126) | | | |
|---|---|---|---|
| | in vitro | in vivo | |
| | $ID_{50}$ (μg/ml) | Dose (mg/kg) | T/C |
| 5a | 0.16 | 50 | 130 |
| 5b | 2.4 | 100 | toxic |
| 5c | 1.7 | | |
| 6a | 0.08 | 30 | 150 |
| 6b | 0.30 | | |

Contrarily to the teachings of the prior art, now it has surprisingly been found that benzo[g]phthalazine-5,10-dione analogues substituted at the 6- and 9-positions by variously substituted amino groups, have a remarkable in vivo antitumor activity.

The compounds of the invention have the following formula (I)

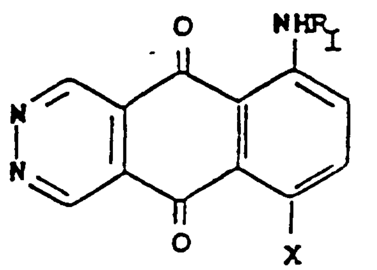

(I)

wherein

X is -OH or $-NHR_2$;

$R_1$ and $R_2$, which can be the same or different, are hydrogen; $(C_1-C_{10})$-alkyl; phenyl; $(C_7-C_{10})$-aralkyl; $(C_2-C_{10})$-alkyl containing at least one of the following substituents: $-O-R_d$;

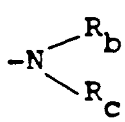 , 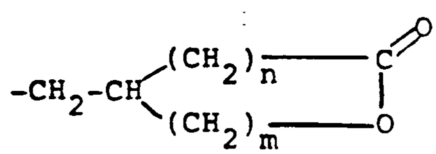

said $(C_2-C_{10})$-alkyl chain being optionally interrupted by one or more oxygen atoms, by one or more groups of formula

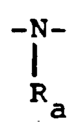

and optionally containing one or more double or triple bonds or hydroxy groups;

$R_a$ is hydrogen; $(C_1-C_{10})$-alkyl; phenyl; $(C_7-C_{10})$-aralkyl; $(C_2-C_{10})$-alkyl substituted with one or more hydroxy groups and/or groups of formula

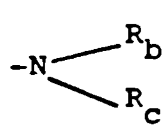 ;

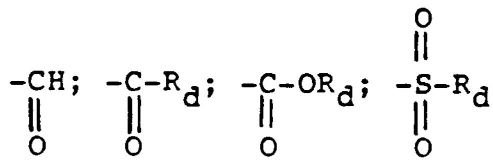

wherein $R_d$ is ($C_1$-$C_{10}$)-alkyl, ($C_7$-$C_{10}$)-aralkyl; phenyl;

$R_b$ and $R_c$, which are the same or different, are hydrogen; ($C_1$-$C_{10}$)-alkyl; ($C_7$-$C_{10}$)-aralkyl; phenyl; ($C_2$-$C_{10}$)-alkyl substituted with one or more hydroxy groups; one of $R_b$ and $R_c$ is a

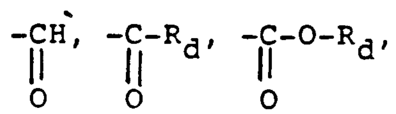

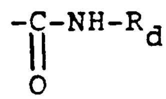

group, $R_d$ being as defined above; or $R_b$ and $R_c$, taken together with the nitrogen atom which they are linked to, form an aziridine ring or a 5-6 membered heterocyclic aromatic or non aromatic ring, optionally containing one or more heteroatoms such as nitrogen, oxygen and sulfur;
n and m are independently zero or the integers 1 and 2, with the proviso that m and n cannot be zero at the same time;
in form of free bases or pharmaceutically acceptable salts thereof.

The present invention also relates to the tautomeric forms, the racemic and diastereoisomeric mixtures of the compounds of formula (I) as well as to the single enantiomers and diastereoisomers thereof.

Examples of pharmaceutically acceptable acids which can salify compounds (I) are inorganic acids, such as hydrochloric, hydrobromic, sulfuric, phosphoric, pyrophosphoric acids or organic acids, such as acetic, propionic, citric, benzoic, lactic, maleic, malic, fumaric, succinic, tartaric, glutamic, aspartic, gluconic, ascorbic acids or other conventionally used acids.

In compounds (I), the words "phenyl" and "5-6 membered heterocyclic ring" mean phenyl rings or heterocyclic rings which can optionally contain substituents such as ($C_1$-$C_4$)-alkyl groups; $CF_3$; halogen atoms; nitro, amino, acetylamino, formylamino, dimethylamino, diethylamino, hydroxy, methoxy and ethoxy groups.

Preferred examples of 5-6 membered aromatic or non aromatic heterocyclic rings containing at least an oxygen or nitrogen atom are, for example, 1-imidazolyl, 1-pyrrolyl, 1-tetrahydropyrrolyl, 1-pyrazolyl, 1-aziridinyl, 4-morpholinyl, 1-piperidinyl, 1-piperazinyl, 1-(4-methyl)piperazinyl, 1-(4-benzyl)piperazinyl, 4-(3-methoxy)morpholinyl, 4-(3-cyano)morpholinyl.

Preferred examples of ($C_7$-$C_{10}$)-aralkyl groups are benzyl and 4-methoxybenzyl.

When in compounds (I) one of $R_1$ and $R_2$ is a straight or branched ($C_2$-$C_{10}$)-alkyl group, optionally interrupted by one or more hetero-atoms, at least 2 carbon atoms are preferably present between said hetero-atoms.

Preferred examples of straight or branched ($C_1$-$C_{10}$)-alkyl groups are methyl, ethyl, n-propyl, sec-propyl, n-butyl, sec-butyl, tert-butyl, n-hexyl, n-pentyl.

Particularly preferred are compounds (I) in which $R_1$ is a substituted ($C_2$-$C_{10}$)-alkyl group, selected from the group consisting of :

- residues of formula $-(CH_2)_p-OH$, wherein p is an integer from 2 to 4;
- residues of formula $-(CH_2)_p-NH_2$ wherein p is as above defined;
- residues of formula $-(CH_2)_p-NH-(CH_2)_q-OH$, wherein p and q are independently an integer from 2 to 4;

- residues of formula

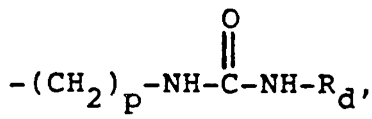

wherein p is as defined above and Rd is ($C_1$-$C_6$)-alkyl or phenyl;

- residues of formula $-(CH_2)_pNR_bR_c$, wherein p is as defined above and $R_b$ and $R_c$ are ($C_1$-$C_6$)-alkyl groups or, taken together with the nitrogen atom, they form an aziridino, pyrrolidino, morpholino, piperazino, N-methylpiperazino, N-benzylpiperazino, piperidino ring;
- residues of formula

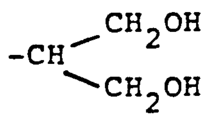 or 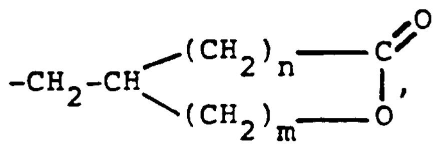

m and n being preferably 1;

- X is OH or $NHR_2$, wherein $R_2$ has the same meanings as $R_1$, defined above.

Specific examples of preferred compounds of the invention are the following ones:
6,9-bis[N-(2-dimethylaminoethyl)amino]benzo[g]phthalazine-5,10-dione;
6,9-bis[N-(2-aminoethyl)amino]benzo[g]phthalazine-5,10-dione;
6,9-bis[N-(3-aminopropyl)amino]benzo[g]phthalazine-5,10-dione;
6,9-bis[N-(4-aminobutyl)amino]benzo[g]phthalazine-5,10-dione;
6,9-bis[N-(2-diethylaminoethyl)amino]benzo[g]phthalazine-5,10-dione;
6,9-bis[N-(2-(2-hydroxyethylamino)ethyl)amino]benzo[g]phthalazine-5,10-dione;
6-[N-(2-(2-hydroxyethylamino)ethyl)amino]-9-[N-(2-dimethylaminoethyl)amino]benzo[g]phthalazine-5,10-dione;
6-[N-(2-(2-hydroxyethylamino)ethyl)amino]-9-[N-(2-aminoethyl)amino]benzo[g]phthalazine-5,10-dione;
6-[N-(1,1-bis(hydroxymethyl)methyl)amino]-9-[N-(2-dimethylaminoethyl)amino]benzo[g]phthalazine-5,10-dione;
6-[N-(2-dimethylaminoethyl)amino]-9-[N-(2-aminoethyl)amino]benzo[g]phthalazine-5,10-dione;
6,9-bis[N-(2-(4-morpholinyl)ethyl)amino]benzo[g]phthalazine-5,10-dione;
6,9-bis[N-(2-(1-pyrrolidinyl)ethyl)amino]benzo[g]phthalazine-5,10-dione;
6,9-bis[N-(2-(1-aziridinyl)ethyl)amino]benzo[g]phthalazine-5,10-dione;
6,9-bis[N-(2-hydroxypropyl)amino]benzo[g]phthalazine-5,10-dione;
6-[N-((2-oxotetrahydzopyran-4-yl)methyl)amino]-9-[N-(2-dimethylaminoethyl)amino]benzo[g]phthalazine-5,10-dione;
6,9-bis[N-[2-(methylcarbamoylamino)ethyl]amino]benzo[g]phthalazine-5,10-dione;
6,9-bis[N-[2-(phenylcarbamoylamino)ethyl]amino]benzo[g]phthalazine-5,10-dione;
6-[N-(2-dimethylaminoethyl)amino]-9-hydroxybenzo[g]phthalazine-5,10-dione;
6-[N-(2-(2-hydroxyethylamino)ethyl)amino]-9-hydroxybenzo[g]phthalazine-5,10-dione;

The compounds of the invention wherein X is the $-NHR_2$ group can be prepared by reacting a compound of formula (II)

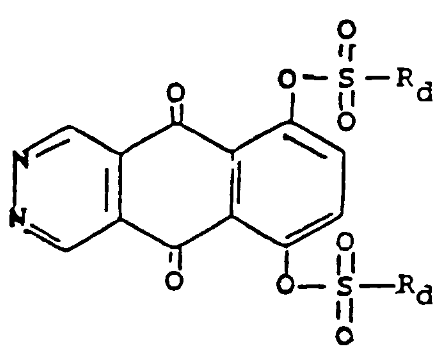

(II)

wherein $R_d$ is as defined above, with a compound of formula (III)

$H_2N-R_x$ (III)

wherein $R_x$ is indifferently one of $R_1$ and $R_2$, or a group which can be converted into $R_1$ and $R_2$ by removing the amino- or hydroxy-protecting groups which can optionally be present, to give a compound of formula (Ia)

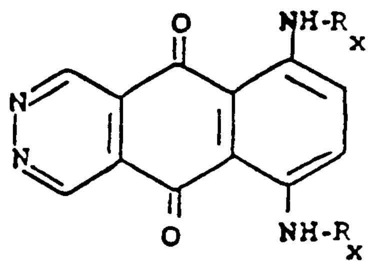

(Ia)

which can subsequently be subjected to one or more of the following reaction steps:

a) when $R_x$ is different from $R_1$ and $R_2$, $R_x$ is transformed into $R_1$ and $R_2$, to give a compound of formula (I);

b) when $R_x$ is one of the groups defined above for $R_1$ and $R_2$, $R_x$ can be converted into another $R_1$ and $R_2$ group to give another compound of formula (I);

c) salification and/or solvation of the obtained compound of formula (I) or separation of the isomers thereof.

Alternatively, compounds (I) wherein $X = NHR_2$ with $R_2$ different from $R_1$, can be prepared by reacting a compound of formula (II) with an equivalent of a compound of formula (IV)

$H_2N-R'_1$ (IV)

wherein $R'_1$ has the same meanings as $R_1$ or it is a group which can be converted into $R_1$ by removing the amino- or hydroxy-protecting groups which are optionally present, to give an intermediate of formula (V)

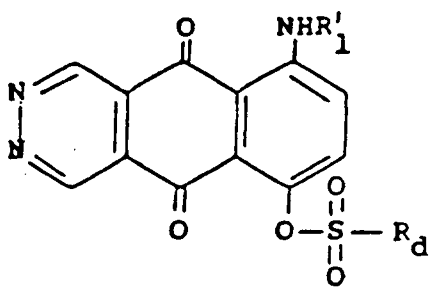

(V)

wherein $R'_1$ and $R_d$ are as above defined, which in its turn can be reacted with a compound of formula (VI)

$H_2N-R'_2$ (VI)

wherein $R'_2$ has the same meanings as $R_2$ or it is a group which can be converted into $R_2$ by removing the amino- or hydroxy-protecting groups which are optionally present, to give an intermediate of formula (Ib)

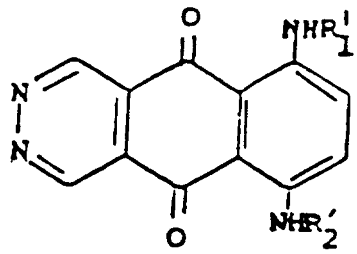

(Ib)

wherein R'$_1$ and R'$_2$ are as above defined, which can be subjected to one or more of the following reaction steps:

a) when either or both of R'$_1$ and R'$_2$ is different from $R_1$ and $R_2$, R'$_1$ and R'$_2$ are transformed into $R_1$ and $R_2$;

b) when R'$_1$ and R'$_2$ are the same as $R_1$ and $R_2$, R'$_1$ and/or R'$_2$ can be converted into another $R_1$ and $R_2$ group, to give another compound of formula (I);

c) salification and/or solvation of the obtained compound of formula (I) or separation of the isomers thereof.

Alternatively, if desired, compounds (I) wherein X is the $-NHR_2$ group, can be prepared by reacting a leuco compound of formula (VII)

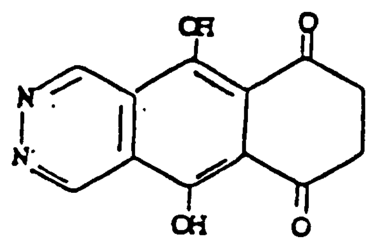

(VII)

with a compound of formula (III), followed by spontaneous oxidation during the working-up of the reaction mixture, to give a compound of formula (Ia) which then can be subjected to one or more of the above described steps, to give a compound of formula (I) wherein X is the $-NHR_2$ group.

Compounds (I) wherein X is the -OH group can be prepared by hydrolysis of intermediate (V) with an alkali or alkaline-earth metal hydroxide, carbonate or hydrogen carbonate, to give a compound of formula (Ic)

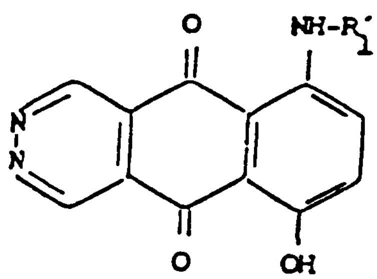

(Ic)

wherein R'$_1$ is as above defined, which compound can be subjected to one or more of the following reaction steps :

a) when R'$_1$ is different from $R_1$, R'$_1$ is transformed into $R_1$, to give a compound (I) wherein X is -OH;

b) when R'$_1$ is the same as $R_1$, R'$_1$ can be converted into another $R_1$ group to give another compound of formula (I) wherein X is -OH;

c) salification and/or solvation of the compound of formula (I) or separation of the isomers thereof.

Compounds of formula (II) can be obtained by the multi-step process shown in scheme I below

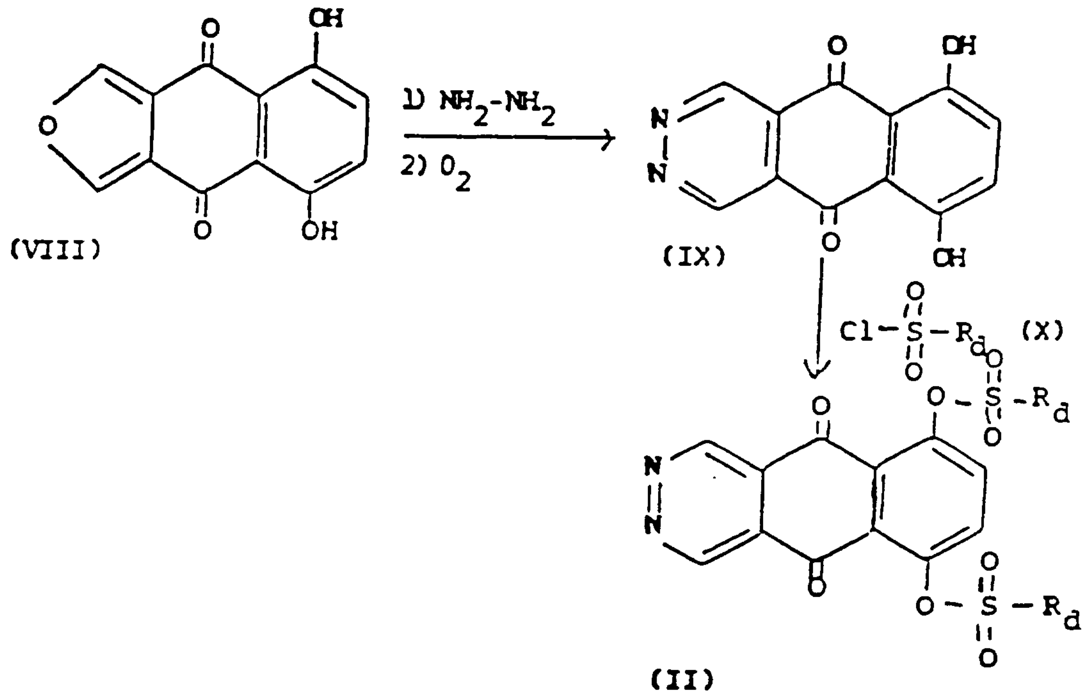


(VIII) (IX) (X) (II)

**Scheme I**

The reaction of compounds (II) with amines (III) can be carried out in the presence of a stoichiometric amount or an excess of compounds (III) in the presence of a solvent such as the compound (III) itself, pyridine, picoline, quinoline, N,N,N',N'-tetramethylethylenediamine and the like, chloroform, methylene chloride, 1,1,1-trichloroethane and the like, optionally in the presence of an inorganic base such as an alkali or alkaline-earth carbonate or hydrogen carbonate or an organic base such as a trialkylamine, e.g. triethylamine, at a temperature from 0 ° C to the reflux temperature of the solvent and for a time ranging from a few hours to some days, depending on the used experimental conditions. Preferably, the reaction is carried out by reacting compounds (II) in the presence of a molar excess of amine (III) in pyridine at room temperature.

If compounds of formula (I) are desired in which X is the -$NHR_2$ group and $R_1$ is different from $R_2$, it is sufficient reacting compounds (II) with at least an equivalent of an amine (IV), to obtain intermediate (V) which can in its turn be reacted with at least one more equivalent of an amine (VI). Also in this case the reaction is generally carried out in pyridine at room temperature.

Reaction of leuco compounds (VII), which can be obtained in situ from compounds (IX) by reduction with a reducing agent such as sodium dithionite in the presence of an inorganic base such as an alkali or alkaline-earth metal hydroxide or carbonate, with amines (III), can be carried out by reacting a stoichiometric amount or an excess of amine (III) in a solvent such as a $C_1$-$C_4$ alcohol, water or mixtures thereof. The reaction can be performed at a temperature from 0 ° C to the reflux temperature of the solvent.

Reaction of intermediate (V) with of an alkali or alkaline-earth metal hydroxide or carbonate, to give a compound of formula (Ic), is generally carried out using a stoichiometric amount or an excess of hydroxide or carbonate, in a solvent such as water, a $C_1$-$C_4$ alcoholor mixtures thereof, at a temperature from 0 ° C to the solvent's reflux temperature.

The reaction is preferably performed in ethanol, using sodium hydroxide, at reflux temperature.

The removal of primary- or secondary-amine protecting groups or hydroxy-protecting groups optionally present in compounds of formulae (Ia), (Ib) or (Ic) can be carried out according to well-known methods, such as by hydrolysis of the esters and amides of compounds of formulae (Ia), (Ib) or (Ic) with organic or inorganic acids or bases, or by selective cleavage of the protecting groups, using suitable reducing and oxidizing agents; the preferred reagent of course depending on the nature of the protecting group.

The multi-step process by which compounds of formula (II) are prepared includes reacting 5,8-dihydroxynaphtho[2,3-c]furane-4,9-dione (VIII) with 80% hydrazine at room temperature, to give 6,9-dihydroxybenzo[g]phthalazine-5,10-dione (IX), which in its turn is reacted with an alkyl, aralkyl or arylsul-

fonyl chloride

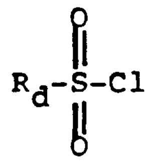

of formula (X), to yield compounds of formula (II).

Reaction of 6,9-dihydroxybenzo[g]phthalazine-5,10-dione (IX) with an alkyl, aralkyl or arylsulfonyl chloride is generally carried out in the presence of a stoichiometric amount or a slight excess of the sulfonyl chloride in a solvent such as chloroform, methylene chloride, 1,1,1-trichloroethane, pyridine, collidine, lutidine and the like, or mixtures thereof, optionally in the presence of an organic or inorganic base, or according to Schotten-Baumann method, at a temperature from room temperature to the reflux temperature of the solvent. The reaction is preferably performed using p-toluenesulfonyl chloride in pyridine at room temperature.

Compound (VIII) is known and can be prepared as described in J. Chem. Res. (M), (1979), 3510-3518.

Compounds (III) are commercially available or they can be prepared according to known methods. Useful teachings for preparing some compounds of formula (III) can be found in J. Org. Chem. (1959), 24, 818-820; J. Med. Chem. (1980), 33, 112-117; J. Med. Chem., (1981), 34, 184-189; Synthetic Comm., (1990), 20(16), 2559-2564.

The compounds of the invention, when administered to animals bearing experimental tumors such as P388 murine leukemia, P388/DOXO doxorubicin-resistant P388 murine leukemia, Lewis lung carcinoma, MXI human mammary carcinoma, LOVO human colorectal tumor, LOVO/DOXO doxorubicin-resistant human colorectal tumor, induce a statistically significant regression in the experimental tumors compared to the control animals treated with the only vehicle (water or physiological saline solution) when the compounds of the invention are administered in the form of pharmaceutically acceptable acid salts, or acid solutions tolerated for the intravenous administration, such as 1% citric acid, 5% lactic acid, when the compounds are used as the free bases). The activity of the induced regression is directly proportional to the used dosage (dose-related pharmacological effect) and to the dosage scheme (e.g. single administration in the day after tumor transplant or repeated administrations at days 3, 5, 9 or 3, 5, 9, 14 after the tumor transplant). The compounds of the invention, when administered at the maximum tolerated doses, induce a higher regression of experimental tumors than that caused by doxorubicin and mitoxantrone, at the maximum tolerated doses, under the same experimental conditions. Particularly, the compounds of the invention are markedly more effective than the control drugs in inhibiting the growth of solid tumors which are doxorubicin- and mitoxantrone-resistant.

Therefore, the available experimental data prove that the compounds of the invention have the following features:

- they have a higher therapeutic index than mitoxantrone, since the range of therapeutically effective tolerated doses in the animal is markedly wider than that of mitoxantrone;
- they are effective in inhibiting growth of hematological and solid experimental tumors, such as murine leukemias, murine lung solid tumors, human mammary and colon solid tumors;
- they are effective against experimental solid tumors which are doxorubicin- and mitoxantrone-resistant (multidrug-resistant).

Therefore, due to these characteristics, the compounds of the invention can be used as the active ingredients in therapeutical compositions for the regression and/or the treatment of tumors in mammals, when administered in amounts ranging from 1 mg to about 0.4 g per kg body weight per day. A preferred dosage regimen for optimum results would be from about 1.0 to about 50 mg per kg body weight per day, using unitary dosages so as to administer from about 70 mg to about 3.5 g of active compound to a subject of about 70 kg body weight, in a 24 hour period. This dosage regimen can be adjusted to provide optimum therapeutical response. For example, several divided doses can be administered according to the requirements of the therapeutical situation. A remarkable practical advantage is that the active compound can be administered by the oral, intravenous, intramuscular or subcutaneous routes.

The compositions of the present invention containing compounds of formula (I) can be used for the treatment of blood cancer diseases such as leukemia or other solid and non-solid tumors such as lung carcinoma, colon carcinoma, melanocarcinoma and mammary tumors. As used herein, by regression or treatment is meant arresting or delaying the growth of the tumor. Suitable administration forms for intravenous injection are physiological sterile solutions. For the intramuscular or intraperitoneal administrations, oily or aqueous injectable preparations are also suitable, whereas suitable forms for the oral administrations are liquid or solid preparations such as syrups, capsules, tablets, and the like.

The invention is further illustrated by the following examples.

## EXAMPLE 1

At 0°C and under magnetic stirring 5,8-dihydroxynaphtho[2,3-c]furane-4,9-dione (2.12 g) is added portionwise during 20 min to 80% hydrazine hydrate (18 ml). The reaction mixture is left under stirring at room temperature for 17 hrs, then it is carefully poured into 60 ml of 6N HCl, while cooling to 0°C. The obtained precipitate is filtered, washed with water, then it is suspended in 40 ml of 1N NaOH. The mixture is heated to 80°C for 15 min, cooled to room temperature and left under stirring under a slight air flow for 18 hrs. Upon addition of concentrated HCl (3.1 ml) and dilution with water (15 ml), a red precipitate is obtained which is filtered, washed first with water, then with some methanol, and finally dried to give 1.98 g of 6,9-dihydroxybenzo[g]phthalazine-5,10-dione; m.p. >220°C, $^{1}$H-NMR ($CDCl_3$)δ: 7,47, s, 2H; 10,07, s, 2H; 12,65, s, 2H, (exchangeable with $D_2O$).

## EXAMPLE 2

Under a nitrogen atmosphere, 4-toluenesulfonyl chloride (2.60 g) is added portionwise during 2 min, to a stirred suspension of 6,9-dihydroxybenzo[g]phthalazine-5,10-dione (1.50 g) in anhydrous pyridine (15 ml) and the obtained mixture is left under stirring at room temperature for 23 hrs. Subsequently the solvent is distilled off under reduced pressure and the obtained residue is purified by column chromatography ($SiO_2$; eluent: dichloromethane/ethyl acetate 20:1) to give yellow-orange crystals of 6,9-bis(4-toluenesulfonyloxy)-benzo[g]phthalazine-5,10-dione (1.57 g); m.p. > 230°C (from $CHCl_3$); $^{1}$H-NMR ($CDCl_3$)δ: 2,47, s, 6H; 7,40, d, 4H; 7,65, s, 2H; 7,90, d, 4H; 9,80, s, 2H.

## EXAMPLE 3

N,N-dimethylethylenediamine (1.0 ml) is added to a solution of 6,9-bis(4-toluenesulfonyloxy)benzo[g]-phthalazine-5,10-dione (0.16 g) in anhydrous pyridine (10 ml) and the resulting mixture is left under stirring at room temperature under a nitrogen atmosphere for 24 hrs. After distilling off the solvent under reduced pressure, the obtained residue is redissolved in $CHCl_3$ (50 ml) and washed with a 3% $NaHCO_3$ solution (2 x 15 ml). The aqueous phases are discarded. The organic phase is dried over $Na_2SO_4$ and, after distilling off the solvent under reduced pressure, the obtained residue is purified by column chromatography ($SiO_2$; eluent: chloroform/methanol/triethylamine 95:4:1) and subsequent recrystallization from dichloromethane/pentane to give blue crystals of 6,9-bis[N-(2-dimethylaminoethyl)amino]benzo[g]phthalazine-5,10-dione, (36 mg); m.p. 188-189°C; $^{1}$H-NMR ($CDCl_3$) : 2,35, s, 12H; 2,68,t, 4H; 3,57, q, 4H; 7,35, s, 2H; 9,95, s, 2H; 11,42, br.t., 2H (exchangeable with $D_2O$); UV (HCl 0,1N): $\lambda_{max}$ (nm), $E_{1\%}$(cm): 245 (475); 279 (337); 351 (106); 684 (337)

H.P.L.C.: Lichrospher 100 RP18 (5 μm); eluent: 10 mM sodium heptanesulfonate + 10 mM $KH_2PO_4$ in water/acetonitrile/dioxane (70:20:10), pH 3 ($H_3PO_4$); eluent flow rate: 1 ml/min; retention time: 4.50 min.

## EXAMPLE 4

Under a nitrogen atmosphere and with stirring a mixture of 6,9-dihydroxybenzo[g]phthalazine-5,10-dione (63 mg), sodium dithionite (97 mg) and anhydrous sodium hydrogen carbonate (17 mg) in anhydrous degased ethanol (7 ml) is heated to reflux for 1.5 hrs. 1-Amino-3-dimethylaminopropane (0.43 ml) is subsequently added and heating is continued for further 12 hours. After distilling off the solvent under reduced pressure, the obtained residue is taken up into chloroform (15 ml) and left under stirring at room temperature, in an open flask, for 30 min. The solvent is distilled off again under reduced pressure and the residue is purified by column chromatography ($SiO_2$; eluent: chloroform/methanol/triethylamine 95:5:1). Blue crystals of 6,9-bis[N-(3-dimethylaminopropyl)amino]benzo[g]phthalazine-5,10-dione (15 mg) are obtained; $^{1}$H-NMR ($CDCl_3$)δ: 2,01, m, 4H; 2,35, s, 12H; 2,55, t, 4H; 3,55, q, 4H; 7,36, s, 2H; 9,96, s, 2H; 11,15, br.t., 2H (exchangeable with $D_2O$).

**EXAMPLE 5**

Under a nitrogen atmosphere N,N-dimethylethylenediamine (0.11 ml) is added to a stirred suspension of 6,9-bis(4-toluenesulfony1oxy)benzo[g]phthalazine-5,10-dione (300 mg) in anhydrous pyridine (5 ml), cooled to 0 ° C. After 6 hrs at 0 ° C, the reaction mixture is allowed to warm to room temperature and after 17 hrs the solvent is distilled off under reduced pressure and the residue is purified by column chromatography ($SiO_2$; eluent: chloroform/methanol 100:5). Purple crystals of 9-[N-(2-dimethylaminoethyl)-amino]-6-(4-toluenesulfonyloxy)benzo[g]phthalazine-5,10-dione (118 mg) are obtained; $^1$H-NMR ($CDCl_3$)δ: 2,45, s, 9H; 2,72, t, 2H; 3,47, q, 2H; 7,17, d, 1H; 7,32, d, 2H; 7,48, d, 1H; 7,85, d, 2H; 9,67, s, 1H; 9,95, s, 1H; 10,25, br.t., 1H.

**EXAMPLE 6**

Following the procedure described in Example 5, the following compounds are prepared:
9-[N-(2-(2-hydroxyethylamino)ethyl)amino]-6-(4-toluenesulfonyloxy)benzo[g]phthalazine-5,10-dione;
9-[N-(2-tert-butoxycarbonylaminoethyl)amino]-6-(4-toluenesulfonyloxy)benzo[g]phthalazine-5,10-dione.

**EXAMPLE 7**

N-(Tert-butoxycarbonyl)ethylenediamine (Synthetic Comm., 20, 2559, (1990)) (340 mg) is added to a suspension of 9-[N-(2-dimethylaminoethyl)amino]-6-(4-toluenesulfonyloxy)benzo[g]phthalazine-5,10-dione (195 mg) in anhydrous pyridine (5 ml) and the resulting mixture is heated to 50 ° C for 8 hrs, under a nitrogen atmosphere, with stirring. The solvent is distilled off under reduced pressure and the residue is purified by column chromatography ($SiO_2$; eluent: chloroform/methanol/triethylamine 95:4:1), to give blue crystals of 6-[N-(2-tert-butoxycarbonylaminoethyl)amino]-9-[N-(2-dimethylaminoethyl)amino]benzo[g]-phthalazine-5,10-dione (120 mg); $^1$H-NMR ($CDCl_3$)δ: 1,53, s, 9H; 2,34, s, 6H; 2,72, t, 2H; 3,51, q, 2H; 3,60÷3,78, m, 4H; 5,60, m, 1H; 7,30, d, 1H; 7,51, d, 1H; 9,85, s, 1H; 9,96, s, 1H; 10,40, br.t., 1H; 10,63, br.t., 1H.

**EXAMPLE 8**

Following the procedure described in Example 3, using N-(tert-butoxycarbonyl)ethylenediamine, blue crystals of 6,9-bis[N-(2-tert-butoxycarbonylaminoethyl)amino]benzo[g]phthalazine-5,10-dione are obtained, m.p. 209-210 ° C; $^1$H-NMR ($CDCl_3$)δ: 1,55, s, 18H; 3,50, q, 4H; 3,70, q, 4H; 5,50, m, 2H (exchangeable with $D_2O$); 7,49, s, 2H; 9,85, s, 2H; 11,65, m, 2H (exchangeable with $D_2O$).

Gaseous HCl is bubbled through a solution of the above compound (245 mg) in anhydrous chloroform (200 ml) until the end of formation of a precipitate, which is filtered under a nitrogen atmosphere, washed with dichloromethane and dried to give 175 mg of 6,9-bis[N-(2-aminoethyl)amino]benzo[g]phthalazine-5,10-dione trihydrochlorido. m.p. > 225 ° C $^1$H-NMR ($D_2O$)δ: 3,35, t, 4H; 3,85, br.m, 4H; 7,40, br.s, 2H; 9,43, s, 2H. U.V. ($H_2O$): $\lambda_{max}$ (nm); $E_{1/0}$ (cm): 245(457); 279(300); 342(95); 669(334).
H.P.L.C.: lichrospher 100 RP 18, eluent: 2 g/l sodium heptanesulfonate in water/acetonitrile/dioxane 75/25/5, pH 3 with $H_3PO_4$; eluent flow rate: 1 ml/min.; λ = 230 nm; retention time: 4.13 min.

**EXAMPLE 9**

A solution of 6.9 N HCl in methanol (0.58 ml) is added to a solution of 6,9-bis[N-(2-dimethylaminoethyl)-amino]benzo[g]phthalazine-5,10-dione (0,38 g) in chloroform (30 ml), while cooling with ice-water. After 30 min the mixture is diluted with diethyl ether (30 ml) and the obtained precipitate is filtered and washed with diethyl ether, to give 6,9-bis[N-(2-dimethylaminoethyl)amino]benzo[g]phthalazine-5,10-dione trihydrochloride (0.35 g).

**EXAMPLE 10**

Following the procedures described in Examples 3, 4, 7 and 8, the following compounds are prepared, which are isolated as the free bases:
6,9-bis[N-(4-aminobutyl)amino]benzo[g]phthalazine-5,10-dione
6,9-bis[N-(2-diethylaminoethyl)amino]benzo[g]phthalazine-5,10-dione
$^1$H-NMR ($CDCl_3$)δ: 1,12, t, 12H; 2,60, q, 8H; 2,74, t, 4H; 3,58, q, 4H; 7,31, s, 2H; 9,55, s, 2H; 11,23, t, 2H;

6,9-bis[N-(2-(2-hydroxyethylamino)ethyl)amino]benzo[g]phthalazine-5,10-dione
$^1$H-NMR ($CDCl_3$)δ: 2,96, t, 4H; 3,07, t, 4H; 3,52, q, 4H; 3,82, q, 4H; 7,05, s, 2H; 9,58, s, 2H; 11,30, br.t., 2H;
6-[N-(2-(2-hydroxyethylamino)ethyl)amino]-9-[N-(2-aminoethyl)amino]benzo[g]phthalazine-5,10-dione
$^1$H-NMR ($CDCl_3$)δ: 2,97, t, 2H; 3,08, t, 2H; 3,17, t, 2H; 3,55, m, 4H; 3,83, t, 2H; 7,02, d, 1H; 7,25, d, 1H; 9,62, s, 1H; 9,80, s, 1H; 11,21, t, 1H; 11,30, t, 1H; 6-[N-(1,1-bis(hydroxymethyl)methyl)amino]-9-[N-(2-dimethylaminoethyl)amino]benzo[g]phthalazine-5,10-dione
$^1$H-NMR ($CDCl_3$)δ: 2,37, s, 6H; 2,70, t, 2H; 3,57, q, 2H; 3,80÷3,92, m, 5H; 7,20, d, 1H; 7,35, d, 1H; 9,65, s, 1H; 9,88, s, 1H;
6,9-bis[N-(2-(4-morpholinyl)ethyl)amino]benzo[g]phthalazine-5,10-dione
$^1$H-NMR ($CDCl_3$)δ: 2,53, m, 8H; 2,78, t, 4H; 3,58, q, 4H; 3,75, m, 8H; 7,29, s, 2H; 9,58, s, 2H; 11,30, br.t., 2H;
6,9-bis[N-(2-(1-pyrrolidinyl)ethyl)amino]benzo[g]phthalazine-5,10-dione
$^1$H-NMR ($CDCl_3$)δ: 1,85, m, 8H; 2,68, m, 8H; 2,88, t, 4H; 3,63, q, 4H; 7,26, s, 2H; 9,55, s, 2H; 11,25, t, 2H;
6,9-bis[N-(2-(1-aziridinyl)ethyl)amino]benzo[g]phthalazine-5,10-dione
$^1$H-NMR ($CDCl_3$)δ: 1,62, s, 8H; 2,60, t, 4H; 3,69, q, 4H; 7,28, s, 2H; 9,57, s, 2H; 11,31, t, 2H;
6,9-bis[N-(3-hydroxypropyl)amino]benzo[g]phthalazine-5,10-dione
$^1$H-NMR ($CDCl_3$)δ: 2,06, m, 4H; 3,58, q, 4H; 3,63, t, 4H; 4,37, br.s., 2H; 7,29, s, 2H; 9,45, s, 2H; 11,60, t, 2H;
6-[N-(2-(2-hydroxyethylamino)ethyl)amino]-9-[N-(2-dimethylaminoethyl)amino]benzo[g]phthalazine-5,10-dione
$^1$H-NMR ($CDCl_3$)δ: 2,36, s, 6H; 2,69, t, 2H; 2,99, m, 2H; 3,10, t, 2H; 3,55, m, 4H; 3,73, t, 2H; 7,28, d, 1H; 7,51, d, 1H; 9,70, s, 1H; 9,93, s, 1H; 10,25, br.t., 1H; 10,51, br.t., 1H;
6,9-bis[N-[3-(tert-butoxycarbonylamino)propyl]amino]benzo[g]phthalazine-5,10-dione
6-[N-(2-dimethylaminoethyl)amino]-9-[N-[2-(tert-butoxycarbonylamino)ethyl]amino]benzo[g]phthalazino-5,10-dione
6,9-bis[N-[2-(methylcarbamoylamino)ethyl]amino]benzo[g]phthalazine-5,10-dione
6,9-bis[N-[2-(phenylcarbamoylamino)ethyl]amino]benzo[g]phthalazine-5,10-dione

## EXAMPLE 11

Following the procedures described in Examples 8 and 9, using the appropriate free bases as the starting products, the following compounds are prepared:
6,9-bis[N-(3-aminopropyl)amino]benzo[g]phthalazine-5,10-dione trihydrochloride
$^1$H-NMR ($D_2O$)δ: 2,25, m, 4H; 3,25, t, 4H; 3,78, t, 4H; 7,30, s, 2H; 9,57, s, 2H;
6,9-bis[N-(4-aminobutyl)amino]benzo[g]phthalazine-5,10-dione trihydrochloride
$^1$H-NMR ($D_2O$)δ: 2,15, m, 8H; 3,27, t, 4H; 3,76, t, 4H; 7,28, s, 2H; 9,58, s, 2H;
6-[N-(2-dimethylaminoethyl)amino]-9-[N-(2-aminoethyl)amino]benzo[g]phthalazine-5,10-dione trihydrochloride
$^1$H-NMR ($D_2O$)δ: 2,60, s, 6H; 2,90, t, 2H; 3,31, t, 2H; 3,85, m, 4H; 7,32, d, 1H; 7,43, d, 1H; 9,50, s, 1H; 9,71, s, 1H.

## EXAMPLE 12

20% NaOH (0.1 ml) is added to a solution of 9-[N-(2-dimethylaminoethyl)amino]-6-(4-toluenesulfonyloxy)benzo[g]phthalazine-5,10-dione (46 mg) in 95° ethanol (2 ml) and the obtained mixture is heated to 50°C for 4 hrs. After cooling to room temperature, the reaction mixture is concentrated to small volume, neutralized with 1N HCl, saturated with sodium chloride and extracted with chloroform (5 x 4 ml). The combined extracts are dried, the solvent is distilled off under reduced pressure and the obtained residue is purified by column chromatography (silica gel; eluent: chloroform/methanol 95/5) to give 6-[N-(2-dimethylaminoethyl)amino]-9-hydroxybenzo[g]phthalazine-5,10-dione

## EXAMPLE 13

Following the procedure described in Example 12, 6-[N-(2-(2-hydroxyethylamino)ethyl)amino]-9-hydroxybenzo[g]phthalazine-5,10-dione is prepared.

## Claims

1. Compounds of formula (I)

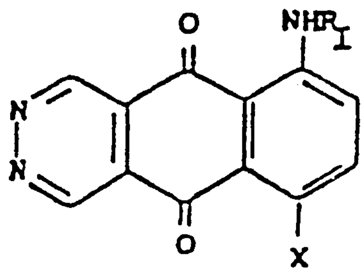

(I),

wherein

X is -OH or -$NHR_2$;

$R_1$ and $R_2$, which can be the same or different, are hydrogen; ($C_1$-$C_{10}$)-alkyl; phenyl; ($C_7$-$C_{10}$)-aralkyl; ($C_2$-$C_{10}$)-alkyl containing at least one of the following substituents: -O-$R_d$;

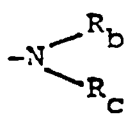 , 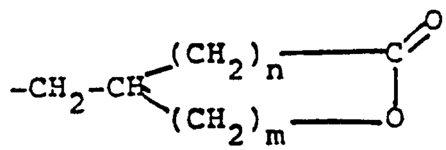

said ($C_2$-$C_{10}$)-alkyl chain being optionally interrupted by one or more oxygen atoms, by one or more groups of formula

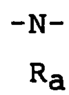

and optionally containing one or more double or triple bonds or hydroxy groups;

$R_a$ is hydrogen; ($C_1$-$C_{10}$)-alkyl; phenyl; ($C_7$-$C_{10}$)-aralkyl; ($C_2$-$C_{10}$)-alkyl substituted with one or more hydroxy groups and/or groups of

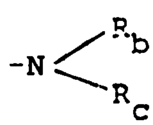 ;

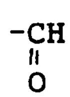 ; 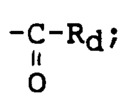 ; 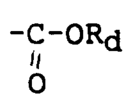 ; 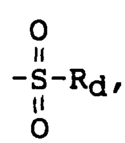 ,

wherein $R_d$ is ($C_1$-$C_{10}$)-alkyl, ($C_7$-$C_{10}$)-aralkyl; phenyl;

$R_b$ und $R_c$, which are the same or different, are hydrogen; ($C_1$-$C_{10}$)-alkyl; ($C_7$-$C_{10}$)-aralkyl; phenyl; ($C_2$-$C_{10}$)-alkyl substituted with one or more hydroxy groups; one of $R_b$ and $R_c$ is a

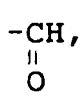 , 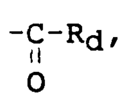 , 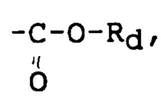 ,

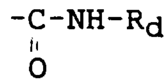

group, $R_d$ being as defined above; or $R_b$ and $R_c$, taken together with the nitrogen atom which they are linked to, form an aziridine ring or 5-6 membered heterocyclic aromatic or non aromatic ring, optionally containing one or more heteroatoms, such as nitrogen and oxygen;
n and m are independently zero or the integers 1 and 2, with the proviso that m and n cannot be zero at the same time;
where phenyl and 5-6 membered heterocyclic rings can optionally contain substituents such as ($C_1$-$C_4$)-alkyl groups; $CF_3$; halogen atoms; nitro, amino, acetylamino, formylamino, dimethylamino, diethylamino, hydroxy, methoxy and ethoxy groups;
in form of free bases or pharmaceutically acceptable salts thereof.

**2.** Compounds as claimed in claim 1, wherein $R_1$ is a substituted ($C_2$-$C_{10}$)-alkyl group, selected from the group consisting of:

- residues of formula $-(CH_2)_p-OH$, wherein p is an integer 2 to 4;
- residues of formula $-(CH_2)_p-NH_2$, wherein p is as above defined;
- residues of formula $(CH_2)_p-NH-(CH_2)_q-OH$, wherein p and q are independently an integer 2 to 4;
- residues of formula

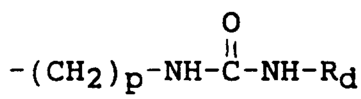 ,

wherein p is as defined above and $R_d$ is ($C_1$-$C_6$)-alkyl or phenyl which can optionally contain substituents such as ($C_1$-$C_4$)-alkyl groups; $CF_3$; halogen atoms; nitro, amino, acetylamino, formylamino, dimethylamino, diethylamino, hydroxy, methoxy and ethoxy groups;
- residues of formula $-(CH_2)_p-NR_bR_c$, wherein p is as defined above and $R_b$ and $R_c$ are ($C_1$-$C_6$)-alkyl groups or, taken together with the nitrogen atom, they form an aziridino, pyrrolidino, morpholino, piperazino, N-methylpiperazino, N-benzylpiperazino, piperidino ring;
- residues of formula

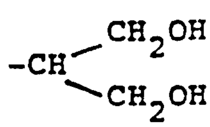 or 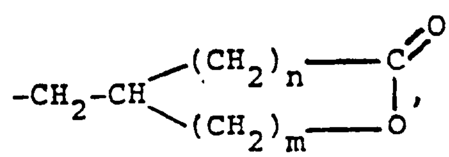 ,

m or n being preferably 1.

**3.** Compounds as claimed in claims 1 and 2, wherein X is $NHR_2$.

**4.** Compounds as claimed in claims 1 and 2, wherein X is hydroxy.

**5.** A process for the preparation of compounds (I), wherein X is $NHR_2$, which process comprises reacting a compound of formula (II)

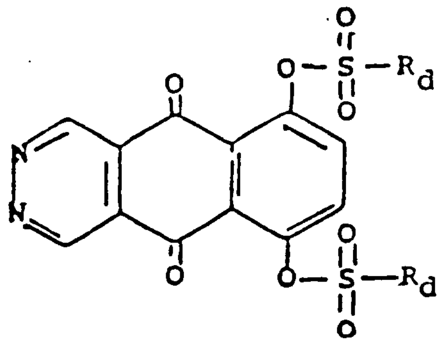

(II)

wherein Rd is as defined above, with a compound of formula (III)

$H_2N\text{-}R_x$ (III)

wherein $R_x$ is indifferently one of $R_1$ and $R_2$, or a group which can be converted into $R_1$ and $R_2$ by removing the amino- or hydroxy-protecting groups which can optionally be present, to give a compound of formula (Ia)

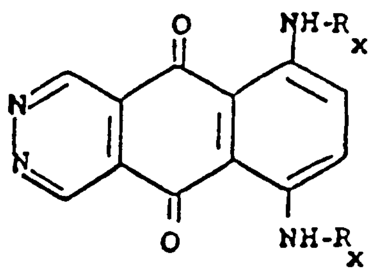

(Ia)

which can subsequently be subjected to one or more of the following reaction steps:

a) when $R_x$ is different from $R_1$ and $R_2$, $R_x$ is transformed into $R_1$ and $R_2$, to give a compound of formula (I);

b) when $R_x$ is one of the groups defined above for $R_1$ and $R_2$, $R_x$ can be converted into another $R_1$ and $R_2$ group to give another compound of formula (I);

c) salification and/or solvation of the obtained compound of formula (I) or separation of the isomers thereof.

**6.** A process for the preparation of compounds (I), wherein X is $NHR_2$ and $R_2$ is different from $R_1$, which process comprises reacting a compound of formula (II) with an equivalent of a compound of formula (IV)

$H_2N\text{-}R'_1$ (IV)

wherein $R'_1$ has the same meanings as $R_1$ or it is a group which can be converted into $R_1$ by removing the amino- or hydroxy-protecting groups which are optionally present, to give an intermediate of formula (V)

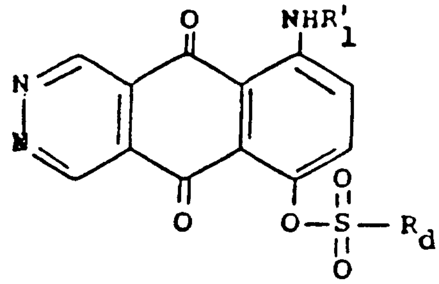

(V)

wherein $R'_1$ and $R_d$ are as above defined, which in its turn can be reacted with a compound of formula (VI)

$H_2N\text{-}R'_2$ (VI)

wherein $R'_2$ has the same meanings as $R_2$ or it is a group which can be converted into $R_2$ by removing the amino- or hydroxy-protecting groups which are optionally present, to give an intermediate of formula (Ib)

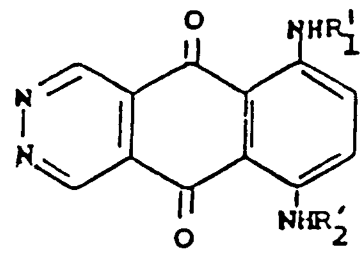

(Ib)

wherein $R'_1$ and $R'_2$ are as above defined, which can be subjected to one or more of the following reaction steps:

a) when either or both of $R'_1$ and $R'_2$ is different from $R_1$ and $R_2$, $R'_1$ and $R'_2$ are transformed into $R_1$ and $R_2$;

b) when $R'_1$ and $R'_2$ are the same as $R_1$ and $R_2$, $R'_1$ and/or $R'_2$ can be converted into another $R_1$ and $R_2$ group, to give another compound of formula (I);

c) salification and/or solvation of the obtained compound of formula (I) or separation of the isomers thereof.

**7.** A process for the preparation of compounds (I) wherein X is $-NHR_2$, which process comprises reacting a leuco compound of formula (VII)

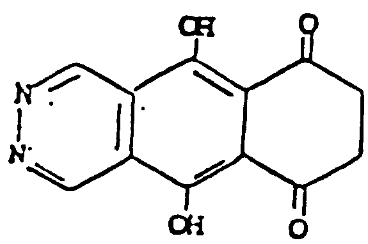

(VII)

with a compound of formula (III), followed by spontaneous oxidation during the working-up of the reaction mixture, to give a compound of formula (Ia) which then can be subjected to one or more of the following steps:

a) when $R_x$ is different from $R_1$ and $R_2$, $R_x$ is transformed into $R_1$ and $R_2$, to give a compound of formula (I);

b) when $R_x$ is one of the groups defined above for $R_1$ and $R_2$, $R_x$ can be converted into another $R_1$ and $R_2$ group to give another compound of formula (I);

c) salification and/or solvation of the obtained compound of formula (I) or separation of the isomers thereof.

**8.** A process for the preparation of compounds (I), wherein X is OH, in which process a compound of formula (V)

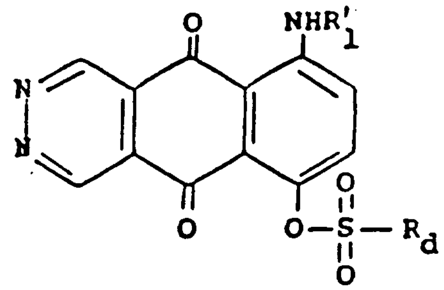

(V)

is hydrolyzed to compound (Ic)

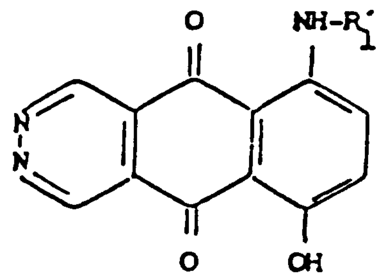

(Ic)

wherein $R'_1$ is as above defined, which compound can be subjected to one or more of the following reaction steps :

a) when $R'_1$ is different from $R_1$, $R'_1$ is transformed into $R_1$, to give a compound (I) wherein X is -OH;

b) when $R'_1$ is the same as $R_1$, $R'_1$ can be converted into another $R_1$ group to give another compound of formula (I) wherein X is -OH;

c) salification and/or solvation of the compound of formula (I) or separation of the isomers thereof.

**9.** Pharmaceutical compositions containing as the active ingredient one compound of claims from 1 to 4, in admixture with suitable excipients.

**10.** The use of the compounds as claimed in claims 1-4 for the preparation of an antitumor medicament.

**Patentansprüche**

**1.** Verbindungen der Formel (I)

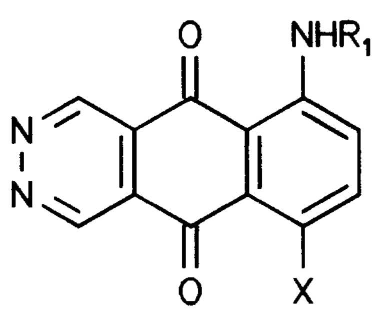

(I)

worin

X -OH oder $-NHR_2$ ist;

$R_1$ und $R_2$, die gleich oder verschieden sein können, Wasserstoff sind; ($C_1$-$C_{10}$)-Alkyl; Phenyl; ($C_7$-$C_{10}$)-Aralkyl;

($C_2$-$C_{10}$)-Alkyl enthaltend wenigstens einen der folgenden Substituenten:

$-O-R_a$;

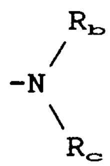 , 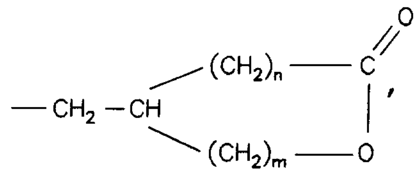 ,

wobei die ($C_2$-$C_{10}$)-Alkyl-Kette gegebenenfalls unterbrochen ist durch ein oder mehrere Sauerstoff-Atome, durch eine oder mehrere Gruppen der Formel

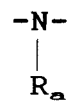

und gegebenenfalls eine oder mehrere Doppel- oder Dreifachbindungen oder Hydroxy-Gruppen enthält;

$R_a$ ist Wasserstoff; ($C_1$-$C_{10}$)-Alkyl; Phenyl; ($C_7$-$C_{10}$)-Aralkyl; ($C_2$-$C_{10}$)-Alkyl, das substituiert ist mit einer oder mehreren Hydroxy-Gruppen und/oder Gruppen der Formel

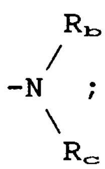 ; 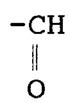 ; 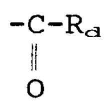 ; 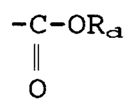 ; 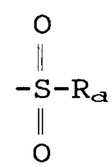 ,

worin $R_d$ ($C_1$-$C_{10}$)-Alkyl, ($C_7$-$C_{10}$)-Aralkyl; Phenyl ist;

$R_b$ und $R_c$, die gleich oder verschieden sind, sind Wasserstoff; ($C_1$-$C_{10}$)-Alkyl; ($C_7$-$C_{10}$)-Aralkyl; Phenyl; ($C_2$-$C_{10}$)-Alkyl, das substituiert ist mit einer oder mehreren Hydroxy-Gruppen;

eines von $R_b$ und $R_c$ ist eine

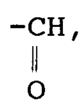 , 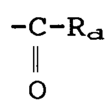 , 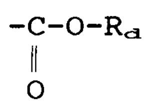 ,

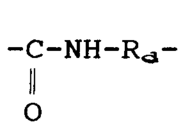

wobei $R_d$ wie vorstehend definiert ist;

oder $R_b$ und $R_c$ bilden zusammen mit dem Stickstoff-Atom, woran sie geknüpft sind, einen Aziridin-Ring oder einen 5- bis 6gliedrigen heterocyclischen aromatischen oder nichtaromatischen Ring, gegebenenfalls enthaltend ein oder mehrere Heteroatome wie etwa Stickstoff und Sauerstoff;

n und m sind unabhängig voneinander null oder die ganzen Zahlen 1 und 2, mit der Maßgabe, daß m und n nicht gleichzeitig null sein können;

wobei Phenyl und die 5-6gliedrigen heterocyclischen Ringe gegebenenfalls Substituenten enthalten

können wie etwa ($C_1$-$C_4$)-Alkyl-Gruppen; $CF_3$; Halogenatome; Nitro-, Amino-, Acetylamino-, Formylamino-, Dimethylamino-, Diethylamino-, Hydroxy-, Methoxy- und Ethoxy-Gruppen;
in Form der freien Basen oder deren pharmazeutisch annehmbarer Salze.

**2.** Verbindungen nach Anspruch 1, worin $R_1$ eine substituierte ($C_2$-$C_{10}$)-Alkyl-Gruppe ist, ausgewählt aus der Gruppe bestehend aus:
- Resten der Formel $-(CH_2)_p-OH$, worin p eine ganze Zahl von 2 bis 4 ist;
- Resten der Formel $-(CH_2)_p-NH_2$, worin p wie vorstehend definiert ist;
- Resten der Formel $-(CH_2)_p-NH-(CH_2)_q-OH$, worin p und q unabhängig voneinander eine ganze Zahl von 2 bis 4 sind;
- Resten der Formel

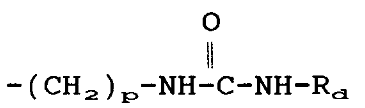,

worin p wie vorstehend definiert ist und $R_d$ ($C_1$-$C_6$)-Alkyl oder Phenyl ist, das gegebenenfalls Substituenten enthalten kann wie etwa ($C_1$-$C_4$)-Alkyl-Gruppen; $CF_3$; Halogenatome; Nitro-, Amino-, Acetylamino-, Formylamino-, Dimethylamino-, Diethylamino-, Hydroxy-, Methoxy- und Ethoxy-Gruppen;
- Resten der Formel $-(CH_2)_pNR_bR_c$, worin p wie vorstehend definiert ist und $R_b$ und $R_c$ ($C_1$-$C_6$)-Alkyl-Gruppen sind oder, zusammengenommen mit dem Stickstoff-Atom, einen Aziridino-, Pyrrolidino-, Morpholino-, Piperazino-, N-Methylpiperazino-, N-Benzylpiperazino-, Piperidino-Ring bilden;
- Resten der Formel

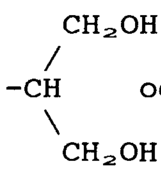 oder 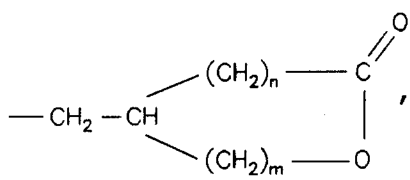,

wobei m und n vorzugsweise 1 sind.

**3.** Verbindungen nach den Ansprüchen 1 und 2, worin X $NHR_2$ ist.

**4.** Verbindungen nach den Ansprüchen 1 und 2, worin X Hydroxy ist.

**5.** Verfahren zur Herstellung von Verbindungen (I), worin X $NHR_5$ ist, umfassend die Umsetzung einer Verbindung der Formel (II)

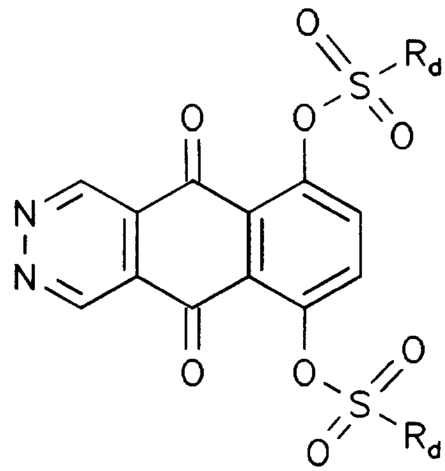

(II)

worin $R_d$ wie vorstehend definiert ist, mit einer Verbindung der Formel (III)

$H_2N-R_x$ (III)

worin $R_x$ eines von $R_1$ und $R_2$ ist oder eine Gruppe, die sich in $R_1$ und $R_2$ überführen läßt durch Abspalten der Amino- oder Hydroxy-schützenden Gruppen, die gegebenenfalls vorhanden sein können, um eine Verbindung der Formel (Ia)

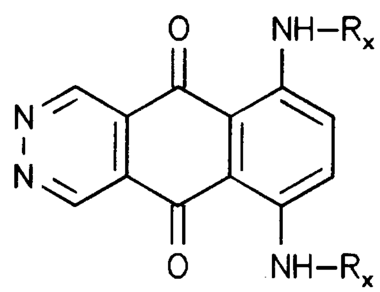

(Ia)

zu ergeben, die anschließend einem oder mehreren der folgenden Reaktionsschritte unterworfen werden kann:

a) ist $R_x$ verschieden von $R_1$ und $R_2$, so wird $R_x$ in $R_1$ und $R_2$ umgewandelt, um eine Verbindung der Formel (I) zu ergeben;

b) ist $R_x$ eine der für $R_1$ und $R_2$ vorstehend definierten Gruppen, kann $R_x$ in eine andere $R_1$- und $R_2$-Gruppe überführt werden, um eine andere Verbindung der Formel (I) zu ergeben;

c) Salzbildung und/oder Solvatation der erhaltenen Verbindung der Formel (I) oder Trennung der Isomere derselben.

**6.** Verfahren zur Herstellung von Verbindungen (I), worin X $NHR_2$ ist und $R_2$ verschieden von $R_1$ ist, umfassend die Umsetzung einer Verbindung der Formel (II) mit einem Äquivalent einer Verbindung der Formel (IV)

$H_2N-R'_1$ (IV)

worin $R'_1$ die gleichen Bedeutungen hat wie $R_1$ oder eine Gruppe ist, die sich in $R_1$ überführen läßt durch Abspaltung der Amino- oder Hydroxy-schützenden Gruppen, die gegebenenfalls vorhanden sind, um ein Zwischenprodukt der Formel (V) zu ergeben,

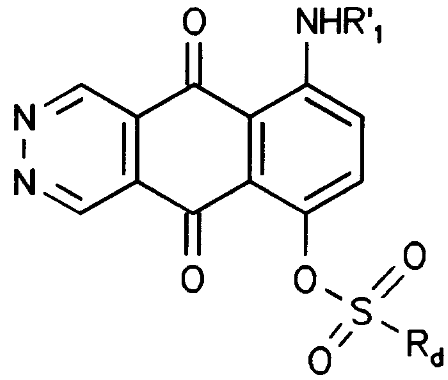

(V)

worin $R'_1$ und $R_d$ wie vorstehend definiert sind, das wiederum mit einer Verbindung der Formel (VI)

$H_2N\text{-}R'_2$ (VI)

umgesetzt werden kann, worin $R'_2$ die gleichen Bedeutungen hat wie $R_2$ oder eine Gruppe ist, die sich in $R_2$ überführen läßt durch Abspaltung der Amino- oder Hydroxy-schützenden Gruppen, die gegebenenfalls vorhanden sind, um ein Zwischenprodukt der Formel (Ib) zu ergeben,

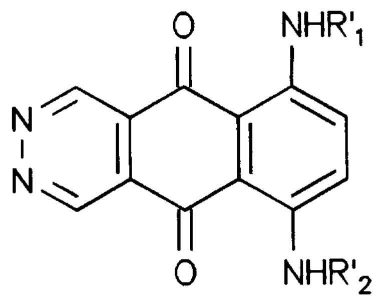

(Ib)

worin $R'_1$ und $R'_2$ wie vorstehend definiert sind, das einem oder mehreren der folgenden Reaktionsschritte unterworfen werden kann:

a) ist eines oder sind beide von $R'_1$ und $R'_2$ verschieden von $R_1$ und $R_2$, so werden $R'_1$ und $R'_2$ in $R_1$ und $R_2$ umgewandelt;

b) sind $R'_1$ und $R'_2$ die gleichen wie $R_1$ und $R_2$, so können $R'_1$ und/oder $R'_2$ in eine andere $R_1$- und $R_2$-Gruppe überführt werden, um eine andere Verbindung der Formel (I) zu ergeben;

c) Salzbildung und/oder Solvatation der erhaltenen Verbindung der Formel (I) oder Trennung der Isomere derselben.

7. Verfahren zur Herstellung von Verbindungen (I), worin X $-NHR_2$ ist, umfassend die Umsetzung einer Leuko-Verbindung der Formel (VII)

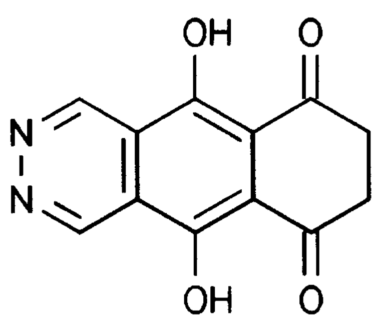

(VII)

mit einer Verbindung der Formel (III), gefolgt von spontaner Oxidation während der Aufarbeitung der Reaktionsmischung, um eine Verbindung der Formel (Ia) zu ergeben, die dann einem oder mehreren der folgenden Schritte unterworfen werden kann:

a) ist $R_x$ verschieden von $R_1$ und $R_2$, so wird $R_x$ in $R_1$ und $R_2$ umgewandelt, um eine Verbindung der Formel (I) zu ergeben;

b) ist $R_x$ eine der für $R_1$ und $R_2$ vorstehend definierten Gruppen, kann $R_x$ in eine andere $R_1$- und $R_2$-Gruppe überführt werden, um eine andere Verbindung der Formel (I) zu ergeben;

c) Salzbildung und/oder Solvatation der erhaltenen Verbindung der Formel (I) oder Trennung der Isomere derselben.

**8.** Verfahren zur Herstellung von Verbindungen (I), worin X OH ist, in dem eine Verbindung der Formel (V)

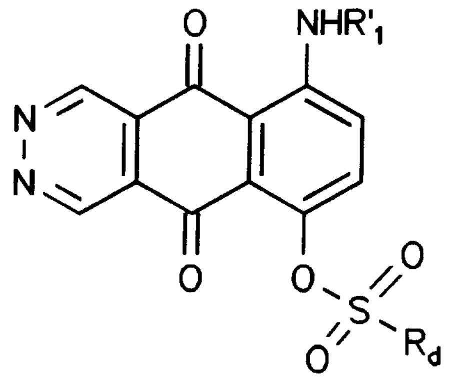

(V)

zur Verbindung (Ic)

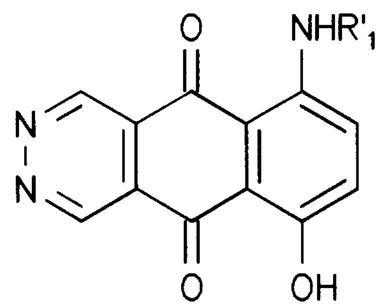

(Ic)

hydrolysiert wird, worin $R'_1$ wie vorstehend definiert ist, welche einem oder mehreren der folgenden Reaktionsschritte unterworfen werden kann:

a) ist $R'_1$ verschieden von $R_1$, so wird $R'_1$ in $R_1$ umgewandelt, um eine Verbindung (I) zu ergeben, worin X -OH ist;

b) ist $R'_1$ das gleiche wie $R_1$, so kann $R'_1$ in eine andere $R_1$- Gruppe überführt werden, um eine andere Verbindung der Formel (I) zu ergeben, worin X -OH ist;

c) Salzbildung und/oder Solvatation der erhaltenen Verbindung der Formel (I) oder Trennung der Isomere derselben.

**9.** Pharmazeutische Zusammensetzungen, enthaltend als wirksamen Bestandteil eine Verbindung nach den Ansprüchen 1 bis 4 im Gemisch mit geeigneten Trägerstoffen.

**10.** Verwendung der Verbindungen nach den Ansprüchen 1 bis 4 zur Herstellung eines Antitumor-Medikaments.

## Revendications

1. Composés de formule (I)

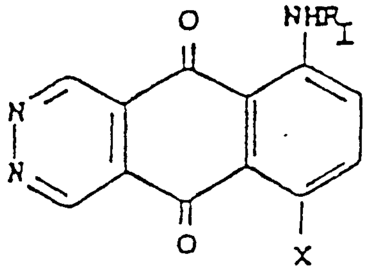

(I),

dans laquelle
X représente -OH ou $-NHR_2$ ;
$R_1$ et $R_2$, qui peuvent être identiques ou différents, représentent un atome d'hydrogène ; un groupe alkyle en $C_1$-$C_{10}$ ; phényle ; aralkyle en $C_7$-$C_{10}$ ; alkyle en $C_2$-$C_{10}$ contenant au moins un des substituants suivants :
$-O-R_d$;

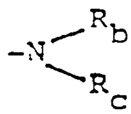 , 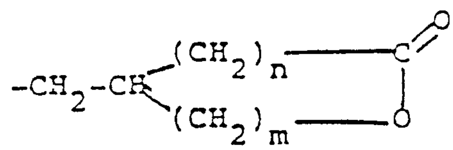

ladite chaîne alkylique en $C_2$-$C_{10}$ étant éventuellement interrompue par un ou plusieurs atomes d'oxygène, un ou plusieurs groupes de formule

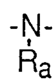

et contenant éventuellement une ou plusieurs liaisons doubles ou triples ou des groupes hydroxyle ;
$R_a$ représente un atome d'hydrogène ; un groupe alkyle en $C_1$-$C_{10}$ ; phényle ; aralkyle en $C_7$-$C_{10}$ ; alkyle en $C_2$-$C_{10}$ substitué par un ou plusieurs groupes hydroxyle et/ou des groupes

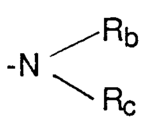 ; 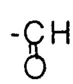 ; 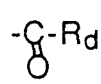 ; 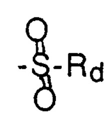 ;

où $R_d$ représente un groupe alkyle en $C_1$-$C_{10}$ ; aralkyle en $C_7$-$C_{10}$ ; phényle ;
$R_b$ et $R_c$, qui sont identiques ou différents, représentent un atome d'hydrogène ; un groupe alkyle en $C_1$-$C_{10}$ ; aralkyle en $C_7$-$C_{10}$ ; phényle ; alkyle en $C_2$-$C_{10}$ substitué par un ou plusieurs groupes hydroxyle ; l'un de $R_b$ et $R_c$ représente un groupe

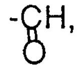, 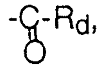, 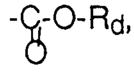, 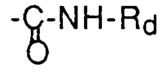 ;

$R_d$ étant défini comme ci-dessus ; ou $R_b$ et $R_c$, pris ensemble avec l'atome d'azote auquel ils sont liés,

forment un cycle aziridine ou un hétérocycle aromatique à 5-6 maillons, ou un cycle non aromatique, contenant éventuellement un ou plusieurs hétéroatomes, tels que l'azote et l'oxygène ;
n et m valent indépendamment zéro ou des nombres entiers de 1 et 2, étant entendu que m et n ne peuvent pas valoir zéro en même temps ;
où le groupe phényle et les hétérocycles à 5-6 maillons peuvent contenir éventuellement des groupes tels que des groupes alkyle en $C_1$-$C_4$ ; $CF_3$ ; des atomes d'halogène ; des groupes nitro, amino, acétylamino, formylamino, diméthylamino, diéthylamino, hydroxy, méthoxy et éthoxy ;
sous la forme de bases libres ou de sels pharmaceutiquement acceptables de ceux-ci.

**2.** Composés selon la revendication 1, où $R_1$ est un groupe alkyle en $C_2$-$C_{10}$, choisi dans le groupe constitué par :

- des résidus de formule $-(CH_2)_p-OH$ où p est un nombre entier de 2 à 4 ;
- des résidus de formule $-(CH_2)_p-NH_2$, où p est défini comme ci-dessus ;
- des résidus de formule $-(CH_2)_p-NH-(CH_2)_q-OH$, où p et q sont indépendamment un nombre entier de 2 à 4 ;
- des résidus de formule

$$-(CH_2)_p-NH-\overset{O}{C}-NH-R_d,$$

  où p est défini comme ci-dessus et $R_d$ est un groupe alkyle en $C_1$-$C_6$ ou phényle, qui peut éventuellement porter des substituants tels que des groupes alkyle en $C_1$-$C_4$ ; $CF_3$ ; des atomes d'halogène ; des groupes nitro, amino, acétylamino, formylamino, diméthylamino, diéthylamino, hydroxy, méthoxy et éthoxy ;
- des résidus de formule $-(CH_2)_p-NR_bR_c$, où p est défini comme ci-dessus et $R_b$ et $R_c$ sont des groupes alkyle en $C_1$-$C_6$ ou, pris ensemble avec l'atome d'azote, ils forment un cycle aziridino, pyrrolidino, morpholino, pipérazino, N-méthylpipérazino, N-benzylpipérazino, pipéridino ;
- des résidus de formule

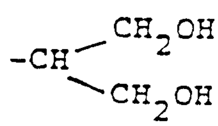 ou 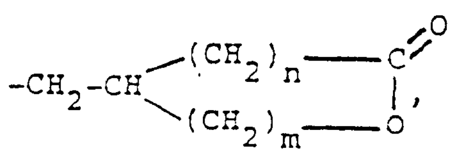

  m et n valant de préférence 1.

**3.** Composés tels que revendiqués dans les revendications 1 et 2, où X représente un groupe $-NHR_2$.

**4.** Composés tels que revendiqués dans les revendications 1 et 2, où X représente un groupe hydroxyle.

**5.** Procédé de préparation de composés de formule (I), dans laquelle X représente un groupe $-NHR_2$, ledit procédé consistant à faire réagir un composé de formule (II)

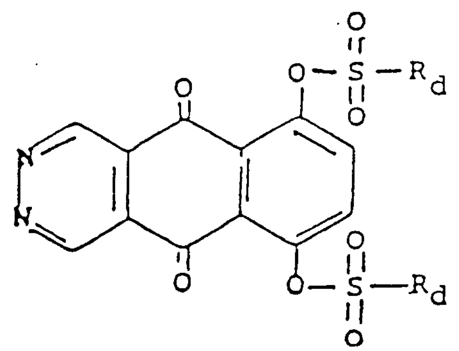

(II)

dans laquelle $R_d$.est défini comme ci-dessus, avec un composé de formule (III)

$H_2N-R_x$. (III)

où $R_x$ représente indifféremment l'un de $R_1$ et $R_2$, ou un groupe qui peut être transformé en $R_1$ et $R_2$ par élimination de groupes protecteurs d'amine ou d'hydroxyle qui peuvent être présents éventuellement, pour donner un composé de formule (Ia)

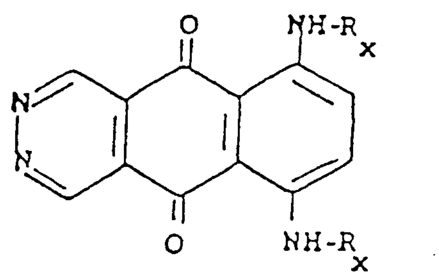

(Ia)

qui peut être soumis par la suite à une ou plusieurs des étapes réactionnelles suivantes :

a) lorsque $R_x$ est différent de $R_1$ et $R_2$, $R_x$ est transformé en $R_1$ et $R_2$ pour produire un composé de formule (I) ;

b) lorsque $R_x$ est l'un des groupes définis ci-dessus pour $R_1$ et $R_2$, $R_x$ peut être transformé en un autre groupe $R_1$ et $R_2$ pour produire un autre composé de formule (I) ;

c) salification et/ou solvatation du composé de formule (I) obtenu ou séparation de ses isomères.

**6.** Procédé de préparation de composés (I), où X représente un groupe $-NHR_2$ et $R_2$ est différent de $R_1$, ledit procédé consistant à faire réagir un composé de formule (II) avec un équivalent d'un composé de formule (IV)

$H_2N-R'_1$. (IV)

dans laquelle $R'_1$ a les mêmes significations que $R_1$ ou il représente un groupe qui peut être transformé en $R_1$ par élimination de groupes protecteurs d'amine ou d'hydroxyle qui sont éventuellement présents, pour donner un composé intermédiaire de formule (V)

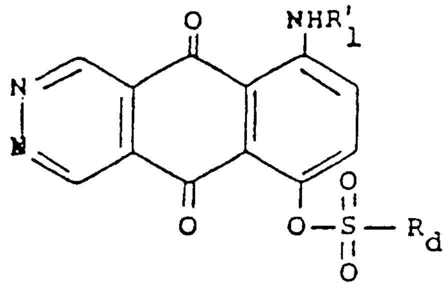

(V)

dans laquelle $R'_1$ et $R_d$ sont définis comme ci-dessus, que l'on peut faire réagir à son tour avec un composé de formule (VI)

$H_2N-R'_2$.    (VI)

dans laquelle $R'_2$ a les mêmes significations que $R_2$ ou il représente un groupe qui peut être transformé en $R_2$ par élimination de groupes protecteurs d'amine ou d'hydroxyle qui sont éventuellement présents, pour donner un composé intermédiaire de formule (Ib)

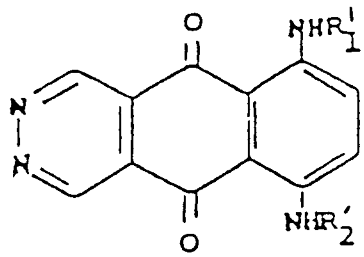

(Ib)

dans laquelle $R'_1$ et $R'_2$ sont définis comme ci-dessus, que l'on peut soumettre à une ou plusieurs des étapes réactionnelles suivantes :

a) lorsque l'un de $R'_1$ et $R'_2$, ou les deux, sont différents de $R_1$ et $R_2$, $R'_1$ et $R'_2$ sont transformés en $R_1$ et $R_2$ ;

b) lorsque $R'_1$ et $R'_2$ sont identiques à $R_1$ et $R_2$, $R'_1$ et/ou $R'_2$ peuvent être transformés en un autre groupe $R_1$ et $R_2$, pour donner un autre composé de formule (I) ;

c) salification et/ou solvatation du composé de formule (I) obtenu ou séparation de ses isomères.

7. Procédé de préparation de composés de formule (I) où X représente un groupe $-NHR_2$, ledit procédé consistant à faire réagir un composé leuco de formule (VII)

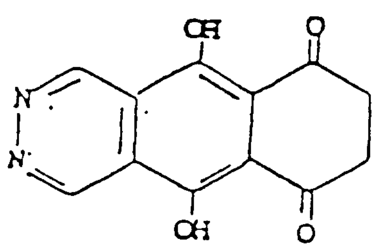

(VII)

avec un composé de formule (III), cette réaction étant suivie d'une oxydation spontanée pendant le traitement ultérieur du mélange réactionnel, pour donner un composé de formule (Ia) qui peut être ensuite soumis à une ou plusieurs des étapes suivantes :

a) lorsque $R_x$ est différent de $R_1$ et $R_2$, $R_x$ est transformé en $R_1$ et $R_2$ pour produire un composé de formule (I) ;

b) lorsque $R_x$ est l'un des groupes définis ci-dessus pour $R_1$ et $R_2$, $R_x$ peut être transformé en un autre groupe $R_1$ et $R_2$ pour produire un autre composé de formule (I) ;

c) salification et/ou solvatation du composé de formule (I) obtenu ou

séparation de ses isomères.

**8.** Procédé de préparation de composés de formule (I), où X représente un groupe OH, procédé dans lequel un composé de formule (V)

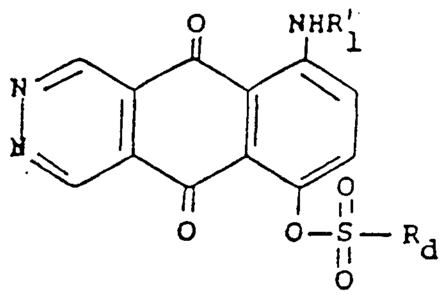

(V)

est hydrolysé en un composé de formule (Ic)

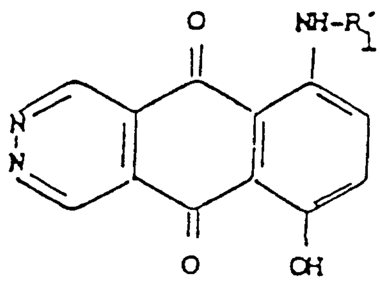

(Ic)

dans laquelle $R'_1$ est défini comme ci-dessus, ledit composé pouvant être soumis à une ou plusieurs des étapes réactionnelles suivantes :

a) lorsque $R'_1$ est différent de $R_1$, $R'_1$ est transformés en $R_1$, pour donner un composé de formule (I) dans laquelle X représente un groupe -OH ;

b) lorsque $R'_1$ est identique à $R_1$, $R'_1$ peut être transformé en un autre groupe $R_1$ pour donner un autre composé de formule (I) dans laquelle X représente un groupe -OH ;

c) salification et/ou solvatation du composé de formule (I) obtenu ou séparation de ses isomères.

**9.** Compositions pharmaceutiques contenant, en tant qu'ingrédient actif, un composé tel que revendiqué dans les revendications 1 à 4, en mélange avec des excipients appropriés.

**10.** Utilisation des composés tels que revendiqués dans les revendications 1 à 4, pour la préparation d'un médicament antitumoral.